# EUROPEAN PATENT APPLICATION

(11) **EP 3 842 073 A1**
(43) Date of publication of application: **30.06.2021**
(21) Application number: 19219453.8
(22) Date of filing: 23.12.2019
(51) Int. Cl.: A61K 41/00, A61K 47/64, A61K 49/00, A61P 35/00

(54) **UPAR-TARGETED PHOTOTHERMAL OPTICAL PROBES FOR PHOTOTHERMAL THERAPY**

(71) Applicant: Rigshospitalet, 2100 Copenhagen Ø (DK)
(72) Inventor: KJÆR, Andreas, 2000 Frederiksberg (DK)
(74) Representative: AWA Sweden AB

(57) **Abstract**

The present invention relates to tumor-targeted probes for use as medicaments and for use in treatment of cancer and to methods of treatment wherein such probes are used. The probes consist of a light-absorbing molecule linked directly or via a spacer to a peptide targeting the urokinase-type plasminogen activator receptor (uPAR). When irradiating the light-absorbing molecule of the probe with laser beams from an external source heat will be released locally to tumor cells expressing uPAR resulting in tumor ablation.

## Description

### FIELD OF THE INVENTION

The present invention relates to tumor-targeted photothermal optical probes for use as medicaments and for use in treatment of cancer and to methods of treatment wherein such probes are used. The probes consist of a light-absorbing molecule linked directly or via a spacer to a peptide targeting the urokinase-type plasminogen activator receptor (uPAR). When irradiating the light-absorbing molecule of the probe with laser beams from an external source heat will be released locally to tumor cells expressing uPAR resulting in tumor ablation. The light-absorbing photothermal molecule of the probe is an optical imageable molecule. The tumor-targeted photothermal optical probe for use as a medicament and specifically for the treatment of cancer may further be combined with positron emission topography (PET) detection targeting the urokinase-type plasminogen activator receptor (uPAR) of tumors and metastases and optical guided surgery, wherein the surgery may be performed prior to activating the tumor-targeted probes by laser irradiation or subsequent to activating the tumor-targeted probes by laser irradiation.

### BACKGROUND OF THE INVENTION

The development of new and more efficient ways to generate localized tumor death without damaging healthy tissue is a need in cancer treatment and management. For this, photothermal cancer therapy (PTT) plays an important role; this therapy relies on photoabsorbing molecules, which are directed to tumor tissue and are able to transform light, such as near-infrared light (NIR), they are irradiated with into heat, causing highly localized tumor death through hyperthermia *(Chatterjee et al., 2011, Day et al., 2011, Riley and Day, 2017).* Nowadays, most photothermal agents are metallic nanoparticles, which present the disadvantage that they are not bio-degradable, remaining in the body for long periods of time *(Jaque et al., 2014).*

The molecule indocyanine green or ICG is a fluorophore with photothermal abilities which is FDA approved and presents valuable imaging properties *(Boni et al., 2015).* Additionally, it has been studied preclinically for its photothermal properties, but in order for ICG to accumulate in the tumor, very high concentrations of the compound (5 mg/kg) are required *(Shirata et al., 2017).* Its instability in water and photobleaching have also limited the use of ICG as a photothermal agent *(Wang et al., 2018).*

Ideally, designing an ICG-loaded nanocarrier or linking the fluorophore to a molecule targeting tumor components would allow for specific accumulation of the molecule in the tumor and more successful treatment outcome *(Wang et al., 2018, Wang et al., 2017, Yan et al., 2016).* For instance, it is known that many tumors express high levels of the urokinase (uPA)-type plasminogen activator receptor (uPAR). When uPA binds to uPAR, a proteolytic cascade is initiated and leads to the destruction of extracellular matrix components. Apart from regulating proteolysis of uPA, uPAR has been studied as a promising therapeutic target in cancer due to its ability to activate multiple intracellular signaling pathways leading to cell adhesion, proliferation and migration. uPAR also plays a role in regulating cancer cell dormancy and angiogenesis *(Montuori et al., 2016, Li et al., 2013).* Importantly, overexpression of uPAR is associated with a more invasive and aggressive cancer progression. Thus, the presence of uPAR in tumors can be used as an advantage for targeted therapies.

Urokinase-type plasminogen activator receptor (uPAR) is frequently over expressed in many cancer types. Expression of uPAR is associated with metastatic disease and poor prognosis and the receptor is often located in excess in the invasive front of the tumour. This makes uPAR ideal as a targeted probe for intraoperative optical imaging. A well validated uPAR targeted peptide AE105 has been used extensively in PET imaging for targeting uPAR previously.

Recently, optical imaging using fluorescence was introduced to help delineating tumors. One example is indocyanin green (ICG) that to some extent unspecifically leaks out into tumors due to vascularization and leaky vessels. However, the unspecific nature of the methods limits its value.

### SUMMARY OF THE INVENTION

It is shown that tumor-targeted photothermal optical probes comprising a light-absorbing molecule linked to a peptide targeting the urokinase-type plasminogen activator receptor (uPAR) can be used as a medicament such as for the treatment of cancer. A method of treating cancer is also described. Apart from the photothermal therapy applicability of the probes, the light-absorbing molecule of the probe is optically imageable. The tumor-targeted photothermal optical probes for use in treatment may further be combined with positron emitting topography diagnosis or surgery or other theranostic methods.

Specifically, the molecule ICG-Glu-Glu-AE105, composed by a uPAR agonist known as AE105 conjugated to ICG, was proven to be a photothermal agent capable of ablating tumor cells. This was tested in a subcutaneous mouse model of human glioblastoma and was surprisingly found to ablate tumors *in vivo.* It was observed that low concentrations of ICG bounded to AE105 was able to specifically and efficiently accumulate in the tumor far better than ICG alone and could cause tumor death when laser-irradiated, leading to a slowdown in tumor growth and an increment in survival in the mice. This ICG-Glu-Glu-AE105 therefore demonstrated that a photothermal uPAR directed agent is an efficient medicament capable of treating cancer after being subjected to irradiation.

Further, the potential of a molecule such as ICG-Glu-Glu-AE105 for molecular imaging-guided photothermal cancer therapy was demonstrated in a subcutaneous mouse model of glioblastoma.

AE105 is a small peptide which acts as uPAR agonist, and together with ICG conforms a fluorescent probe that targets uPAR-expressing tumors and at the same time can be followed throughout the body with fluorescence imaging techniques. The imaging properties of ICG-Glu-Glu-AE105 have previously been described in a preclinical model and have also shown potential for intra-operative optical imaging (Juhl et al., 2016).

It is further shown herein that a probe such as ICG-Glu-Glu-AE105 have other important roles in theranostics by demonstrating the accumulation of the probe in the tumor throughout time using fluorescence imaging.

PTT was performed on subcutaneous glioblastoma tumors in mice and it was observed that the mice which had been injected with ICG-Glu-Glu-AE105 prior to PTT experienced a slowdown in tumor growth after the treatment, as well as an increment in survival compared to mice injected with unconjugated ICG. This proves the value of ICG-Glu-Glu-AE105 as photothermal agent as well as imaging agent, and thus the clinical application as a PTT medicament and specifically for the PPT treatment of cancer of probes comprising a light-absorbing molecule linked to a peptide targeting the urokinase-type plasminogen activator receptor, such as an ICG-linked uPAR agonist.

### BRIEF DESCRIPTION OF THE DRAWINGS

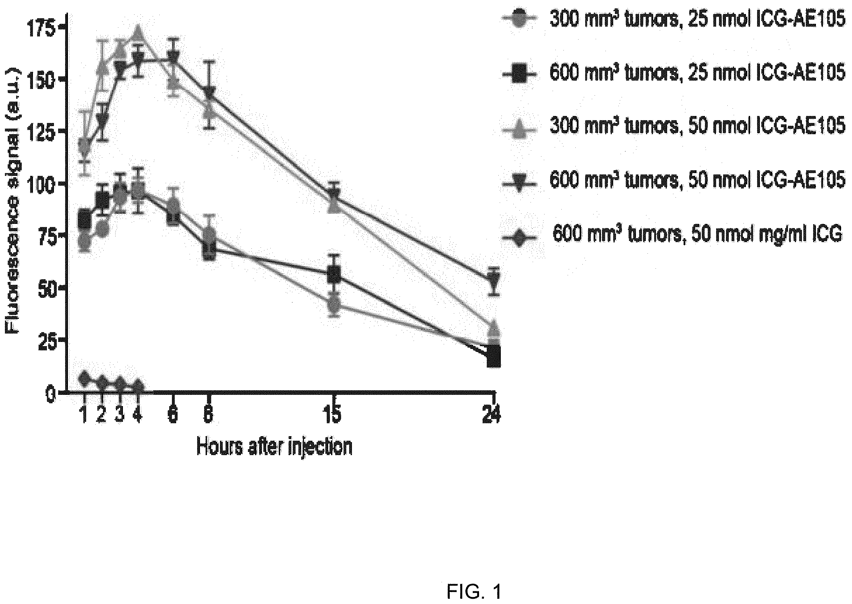

Figure 1: Accumulation of ICG-AE105 and unconjugated ICG in subcutaneous U-87 MG human xenograft tumors. Fluorescence signal emitted by the ICG molecules in either ICG-Glu-Glu-AE105 or ICG alone throughout time, at different concentrations and tumor sizes. n = 3-5 per group. Data shown is mean ± SEM.

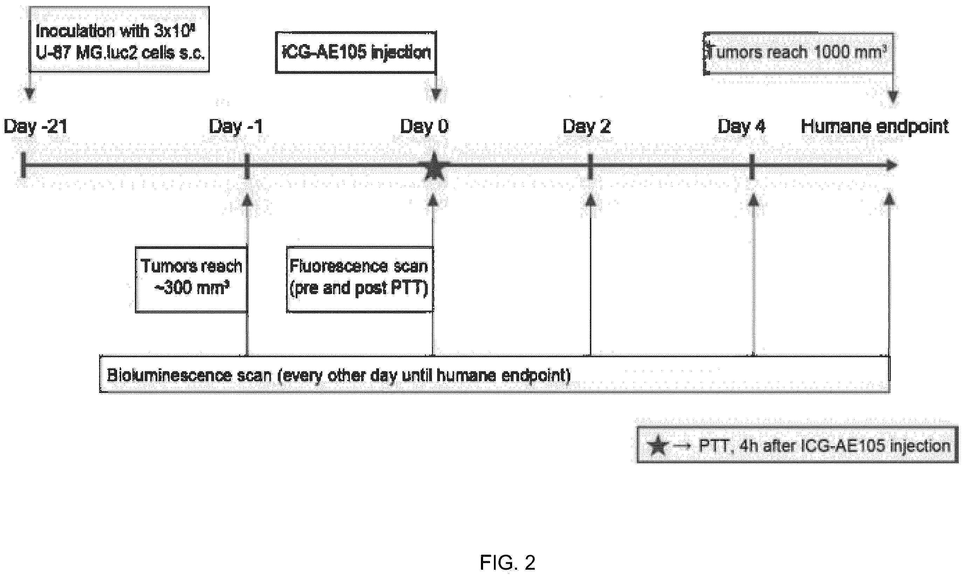

Figure 2: Experimental timeline of the study to test the ICG-Glu-Glu-AE105 ability as a photothermal agent in vivo. Animals were baseline scanned one day before PTT, when tumors reached around 300 mm³, and divided into groups. On day 0, animals were injected with ICG-Glu-Glu-AE105, ICG, or saline four hours before PTT. Afterwards, animals were scanned for bioluminescence every other day until tumors reached 1000 mm³.

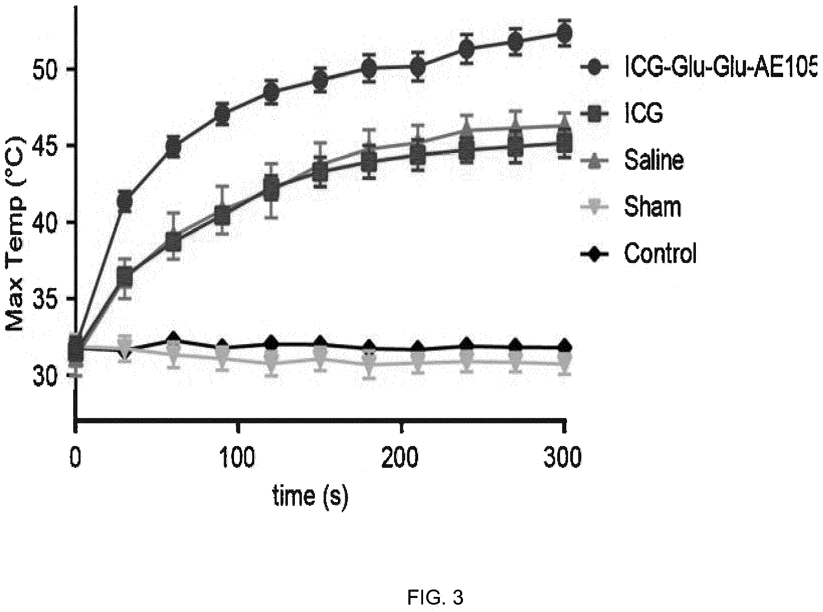

Figure 3: Temperature increase on the tumor surface during PTT. The maximum temperatures reached on the tumor surface for the different groups. Data shown is mean ± SEM. (ICG-Glu-Glu-AE105; n = 6, ICG; n = 4, Saline; n = 4, Sham; n = 5 and Control; n = 4).

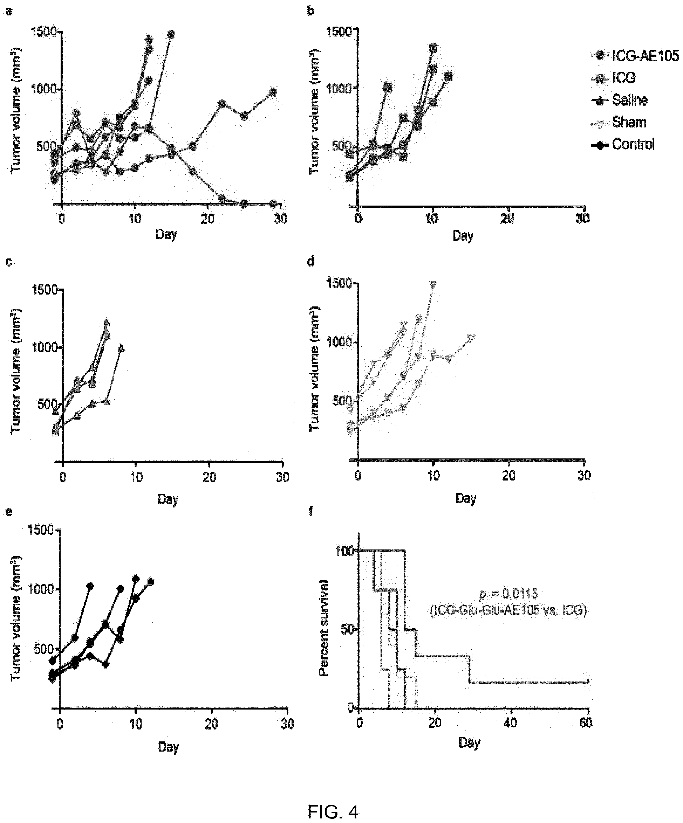

Figure 4: Tumor growth and survival after ICG-Glu-Glu-AE105-based PTT. a-e) Growth curves from the ICG-Glu-Glu-AE105 (n = 6), ICG (n = 4), Saline (n = 4), Sham (n = 5) and Control (n = 4) groups respectively. Curves stopped on day 29 as only one ICG-Glu-Glu-AE105 mouse was left. f) Survival curves for the different groups.

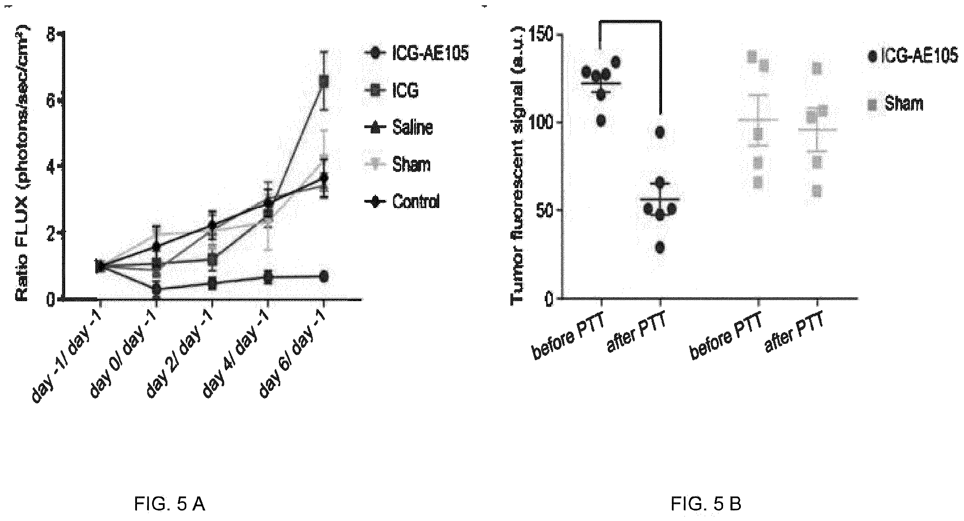

Figure 5: Bioluminescence signal after PTT. 5A: Bioluminescent signal expressed as ratio FLUX (day X/day-1) for the different groups. 5B: Differences in tumor fluorescent signal detected by Fluobeam for ICG-Glu-Glu-AE105 and sham groups.

### DETAILED DESCRIPTION OF THE INVENTION

The uPAR receptor is known to be overexpressed in a variety of cancers, which makes it an attractive target for multiple cancer therapies. Among them, photothermal therapy is a technique which relies on light absorbing agents to cause localized tumor death when irradiated with an external source of light. It is shown herein that use of the molecule ICG-Glu-Glu-AE105, composed by the uPAR agonist AE105, and the fluorophore ICG, is capable of generating heat when irradiated. The fact that ICG is already FDA approved makes this molecule extremely relevant for its use in cancer treatment. It is further shown herein that ICG-Glu-Glu-AE105 is able to efficiently kill subcutaneous tumors through photothermal therapy (PTT) in a mouse model of glioblastoma. Mice bearing subcutaneous U-87MG.luc2 tumors were divided into five groups matching tumor size, and were injected with either ICG-Glu-Glu-AE105, unconjugated ICG, or saline for the three groups undergoing PTT, and two extra control groups (control, no injection, and sham, injected with ICG-Glu-Glu-AE105) which were not irradiated. PTT was performed on the first three groups and bioluminescence scans were carried out every other day to follow tumor growth for all mice. It was found that uPAR-targeted PTT led to a delay in tumor growth and even to complete tumor disappearance for one of the mice. This was not observed for any of the other groups. The delay in tumor growth was also translated into a decrease in bioluminescent signal.

Through immunological staining, uPAR was confirmed to be present throughout all tumors, whereas the ICG-Glu-Glu-AE105 group, due to the high level of tumor death, showed a decrease in uPAR signal.

These results consolidated that tumor-targeted photothermal optical probes comprising a light-absorbing molecule linked to a peptide targeting the urokinase-type plasminogen activator receptor are suitable as medicaments as demonstrated by the ICG-coupled uPAR-targeted probe which has already proven its value as an imaging agent.

Another observation herein is that both *in vitro* and *in vivo* the loss of fluorescent signal after PTT confirmed the degradation of ICG after treatment. This should be taken into account when considering multiple laser treatments.

The experiments described in the Examples were performed in subcutaneous tumors with a volume of around 300 mm³. This was in order to show the potential of the probe and as a proof-of-concept. In a clinical setting, the relevance of these experiments could be translated into surgical procedures, where ICG-Glu-Glu-AE105-based PTT could serve as an adjuvant method that improves clinical outcome.

In one aspect, the present disclosure relates to a probe having the formula X-Y-Z and pharmaceutically acceptable salts thereof, wherein
X represents a photothermic agent capable of absorbing light that is transformed into heat upon irradiation with an external source of light and capable of being detected either directly or indirectly in an optical imaging procedure;
Y represents a spacer or is absent;
and Z is a uPAR targeting peptide;
for use as a medicament subject to irradiation with an external laser source that activates X.

In another aspect, the present disclosure relates to a probe having the formula X-Y-Z and pharmaceutically acceptable salts thereof, wherein
X represents a photothermic agent capable of absorbing light that is transformed into heat upon irradiation with an external laser source and capable of being detected either directly or indirectly in an optical imaging procedure;
Y represents a spacer or is absent;
and Z is a uPAR targeting peptide;
for use in treatment of cancer subject to irradiation with an external laser source.

In one embodiment, X is a photothermic fluorescent agent. The photothermic fluorescent agent may be any photothermic fluorescent agent. Preferably, the fluorophore have a NIR-light absorption in the range of 700-1200 nm, 700-950 nm for NIR-I fluorophores, or 1000-1200 nm for NIR-II fluorophores. In a preferred embodiment, the photothermic fluorescent agent is selected from the group comprising: NIR-I fluorophore selected from the group consisting of ICG, Methylene blue, 5-ALA, Protoporphyrin IX, IRDye800CW, ZW800-1, Cy5, Cy7, Cy5.5, Cy7.5, IRDye700DX, Alexa fluor 488, Fluorescein isothiocyanate.

According to another preferred embodiment of the present invention the fluorophore may be a NIR-II fluorophore selected from the group consisting of Flav7, CH1055, Q1, Q4, H1, IR-FEP, IR-BBEP, IR-E1, IR-FGP, IR-FTAP.

In a preferred embodiment, the photothermic fluorescent agent is indocyanine green (ICG).

Y may be any suitable spacer linking the photothermic agent to the uPAR targeting peptide. For instance, Y may be a linker such as Glu-Glu-O2Oc-O2Oc, a dipeptide such spacer as Glu-Glu, glycyl-glycine (GG), Gly-Gly, Gly-Phe, Phe-Phe, Pro-Pro, or a PEG spacer such as a PEG2 spacer, a PEG3 spacer, a PEG4 spacer, a PEG5 or a PEG6 spacer.

In a preferred embodiment, Y is Glu-Glu. In another preferred embodiment, Y is Glu-Glu-O2Oc-O2Oc. In yet another preferred embodiment, Y is absent.

The uPAR targeting peptide Z may be any peptide that is capable of binding specifically the urokinase-type plasminogen activator receptor (uPAR).

In a preferred embodiment, Z is the uPAR targeting peptide (D-Asp)-([beta]-cyclohexyl-L-alanine)-(Phe)-(D-Ser)-(D-Arg)-(Tyr)-(Leu)-(Trp)-(Ser) which can also be denoted as Asp-Cha-Phe-ser-arg-Tyr-Leu-Trp-Ser, where Cha is β-cyclohexyl-L-alanine. This peptide is also known as AE105. Z may also be the amidated form of AE105: Asp-Cha-Phe-ser-arg-Tyr-Leu-Trp-Ser-NH₂, which is also a preferred embodiment.

In a preferred embodiment, the tumor-targeted photothermal optical probe, is a probe wherein X is ICG, Y is Glu-Glu and Z is (D-Asp)-([beta]-cyclohexyl-L-alanine)-(Phe)-(D-Ser)-(D-Arg)-(Tyr)-(Leu)-(Trp)-(Ser).

In another preferred embodiment, the tumor-targeted photothermal optical probe is a probe wherein X is ICG, Y is Glu-Glu and Z is Asp-Cha-Phe-ser-arg-Tyr-Leu-Trp-Ser-NH2, wherein Cha is β-cyclohexyl-L-alanine.

In another preferred embodiment, the tumor-targeted photothermal optical probe is a probe wherein X is ICG, Y is Glu-Glu-O2Oc-O2Oc and Z is (D-Asp)-([beta]-cyclohexyl-L-alanine)-(Phe)-(D-Ser)-(D-Arg)-(Tyr)-(Leu)-(Trp)-(Ser).

In yet another preferred embodiment, the tumor-targeted photothermal optical probe is a probe wherein X is ICG, Y is Glu-Glu-O2Oc-O2Oc and Z is Asp-Cha-Phe-ser-arg-Tyr-Leu-Trp-Ser-NH2, wherein Cha is β-cyclohexyl-L-alanine.

For instance, the tumor-targeted photothermal optical probe is the preferred probe ICG-Glu-Glu-AE105 as shown below:

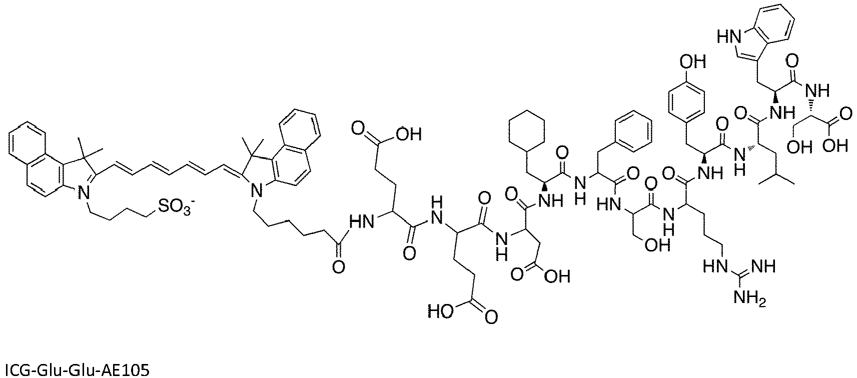

An example of a probe comprising the Glu-Glu-O2Oc-O2Oc linker is shown below:

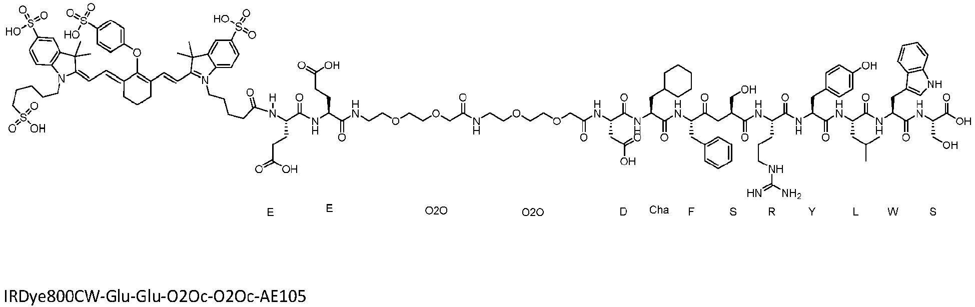

The present tumor-targeted photothermal optical probes for use as a medicament may provide therapeutic effect to any disease wherein ablation of cells expressing urokinase-type plasminogen activator receptor (uPAR) provides a benefit of the subject being treated.

Such diseases includes all cancers wherein uPAR is expressed on the surface of the diseased cells such as solid tumors, leukemia and lymphomas. Preferably, the cancer to be treated is selected from the group comprising: glioblastoma, brain cancer, oropharyngeal cancer, head and neck cancer, prostate cancer, breast cancer, gastro-intestinal cancer, colorectal cancer (CRC), lung cancer, non-small lung cancer (NSCLC).

In preferred embodiments, the tumor-targeted photothermal optical probes are for use as in the treatment of brain cancer or head-and-neck cancer. In a more preferred embodiment the tumor-targeted photothermal optical probes are for use as in the treatment of glioblastoma multiforme.

In one embodiment, X is a fluorescent probe capable of being activated by infrared light at a wavelength between 0.75 - 1000 µm such as near infrared (NIR) light at a wavelength between 0.75 - 3 µm, mid infrared light at a wavelength between 3 - 50 µm or far infrared light at a wavelength between 50 - 1000 µm. Preferably, X have a NIR-light absorption in the range of 700-1200 nm, 700-950 nm for NIR-I fluorophores, or X have a NIR-light absorption in the range of 1000-1200 nm for NIR-II fluorophores.

In one embodiment, the tumor-targeted photothermal optical probe for use in the treatment of cancer is used in combination with optical imaging of X.

In another embodiment, the tumor-targeted photothermal optical probe for use in the treatment of cancer is used in combination with surgery based on the optical imaging of X.

In another embodiment, the tumor-targeted photothermal optical probe for use in the treatment of cancer is used in combination with surgery based on positron emitting topography of an uPAR directed target.

The associated optical imaging of X or positron emitting topography of an uPAR directed target may be applied for the purpose of providing a diagnosis, for optical guided surgery or both. The associated imaging and/or surgery may be applied prior to irradiation of the tumor-targeted photothermal optical probe with an external laser source or after irradiation of the tumor-targeted photothermal optical probe with an external laser source or both prior to and subsequent to irradiation of the tumor-targeted photothermal optical probe with an external laser source.

The tumor-targeted photothermal optical probes may be administered to a subject such as a human or an animal. In a preferred embodiment, the subject is a human. The tumor-targeted photothermal optical probes may be administered to animals such as mammals including but not limited to dogs, cats, horses, monkeys, mice, rats and rabbits.

In one embodiment the uPAR-targeted photothermal optical probes are provided in a pharmaceutical composition for treatment of cancer and optical imaging, wherein the composition comprises a compound of the invention together with at least one pharmaceutically acceptable carrier or excipient. The uPAR-targeted photothermal optical probes may be administered to the subject, such as a human, in a dose of 0.1 - 1000 mg per 70 kg.

In another aspect, the present disclosure relates to a method of treating cancer comprising
(i) administering a probe having the formula X-Y-Z or pharmaceutically acceptable salts thereof, wherein
   X represents a photothermic agent capable of absorbing light that is transformed into heat upon irradiation with an external laser source and capable of being detected either directly or indirectly in an optical imaging procedure;
   Y represents a spacer or is absent;
   and Z is a uPAR targeting peptide;
   to a subject
(ii) subjecting the subject to irradiation with an external laser source that activates X.

In one embodiment, the method of treatment further comprises one or more additional steps carried out either before or after irradiating the subject treated with an external laser source that activates X:
(iii) optical imaging of X;
(iv) optical guided surgery based on the imaging of X;
(v) optical guided surgery based on PET imaging of a uPAR directed target;
(vi) identification and diagnostics based on PET imaging of a uPAR directed target.

In a preferred embodiment of the methods described herein, X is ICG, Y is Glu-Glu, and Z is (D-Asp)-([beta]-cyclohexyl-L-alanine)-(Phe)-(D-Ser)-(D-Arg)-(Tyr)-(Leu)-(Trp)-(Ser).

In another preferred embodiment of the methods described herein, X is ICG, Y is Glu-Glu, and Z is Asp-Cha-Phe-ser-arg-Tyr-Leu-Trp-Ser-NH₂, wherein Cha is β-cyclohexyl-L-alanine.

In another preferred embodiment of the methods described herein, X is ICG, Y is Glu-Glu-O2Oc-O2Oc, and Z is (D-Asp)-([beta]-cyclohexyl-L-alanine)-(Phe)-(D-Ser)-(D-Arg)-(Tyr)-(Leu)-(Trp)-(Ser).

In yet another preferred embodiment of the methods described herein, X is ICG, Y is Glu-Glu-O2Oc-O2Oc, and Z is Asp-Cha-Phe-ser-arg-Tyr-Leu-Trp-Ser-NH₂, wherein Cha is β-cyclohexyl-L-alanine.

All the embodiments including the preferred embodiments mentioned above in relation to the uPAR-targeted photothermal optical probes for photothermal therapy such as treatment of cancer are also embodiments of the methods of treatment.

### EXAMPLES

The following materials and methods were applied in Examples 1 to 4 as described further below.

### In vitro experiments

The ability of ICG to generate high temperatures when irradiated with NIR light was measured in vitro. Different concentrations (228, 46, 23 and 11 nmol) of unconjugated ICG and ICG-Glu-Glu-AE105 in solution (0.2 g/ml (2-hydroxypropyl)-β-cyclodextrin in water and DMSO) were placed in a plastic cuvette under the laser beam. The laser was turned on for ten minutes at 2 W/cm2 and the temperature measured with a NIR camera.

### Cell line and animal model

The animal experiments were approved by the Danish Animal Welfare Council, Ministry of Justice. For the in vivo studies, U-87 MG.luc2 cells were grown in DMEM + P/S + FBS media. When cells reached 70% confluence, they were harvested and 2-3x106 cells in 100µl of PBS were inoculated into the left flank of NMRI nude female mice (Janvier Labs, France). Animals were left to grow tumors until they reached 300 mm³ (day -1).

### ICG-Glu-Glu-AE105 accumulation in vivo

Animals were injected with either ICG-Glu-Glu-AE105 (23 or 46 nmol) or ICG alone (46 nmol) in 200 µl of 0.2 g/ml (2-hydroxypropyl)-β-cyclodextrin in water and 2% DMSO as described before (Juhl et al., 2016). The mice were scanned for fluorescence on the Fluobeam at different time points (1, 2, 3, 4, 6, 8, 15 and 24h). For this, they were kept under anesthesia by breathing 3-5% sevoflurane (Abbott Scandinavia AB, Sweden) mixed with 5% O₂ in N₂ and placed below the optical beam. The exposure time was 20 ns.

### In vivo PTT

When tumors reached 300 mm³, mice were divided into 5 groups: ICG-Glu-Glu-AE105 group (mice injected with 46 nmol ICG-Glu-Glu-AE105 four hours before PTT, n = 6); ICG group (mice injected with 46 nmol of unconjugated ICG four hours before PTT, n = 4); Saline group (mice injected with saline four hours before PTT, n = 4); sham group (mice injected with ICG-Glu-Glu-AE105 and no PTT, n = 5) and a control group (no injection, no PTT, n = 4). Animals were given analgesia (temgesic, 0.3mg/ml) right before the laser treatment and every 6 to 8 hours until deemed necessary. For PTT, animals were anesthetized with sevoflurane as previously described and placed under the laser beam for five minutes at 2 W/cm². The tumors were swabbed with glycerol, an index-matching agent, prior to the laser treatment to facilitate light penetration (Deng et al. 2011, Vargas et al., 1999, Simón et al., 2019). The temperatures reached on the tumor surface were detected with a NIR camera, as described for the in vitro experiments.

### Fluorescence and bioluminescence imaging

Animals were bioluminescence-scanned a day before PTT, on PTT day and afterwards every other day until endpoints were reached. For these bioluminescence scans, luciferin was injected intraperitoneally (5 µl/mg) ten minutes before the scan.

The fluorescence scans were performed to detect ICG signal as described for the accumulation study.

### Histological analysis

Tumors were resected from n = 2 mice per group two days after PTT. Tumors were fixated in formaldehyde overnight, and afterwards conserved in ethanol for their later embedding in paraffin. To perform the histology experiments, tumors were resected in 4µm slices in a microtome (Thermo Scientific Microm HM355S). The tissue slides were left to dry for at least 1.5 hours, and then heated first at 40 and after at 60 °C. Following, slides were submerged in HistoClear to achieve deparaffinization and rehydrated through a series of EtOH. For hematoxylin and eosin staining, the tissue slides were stained with hematoxylin for 5 minutes, rinsed and stained with eosin for 3 minutes.

For immunohistochemistry, the rehydrated tissue slides underwent heat-induced epitope retrieval (HIER) by covering the tissue slides with 250 mL citrate buffer (pH = 6), and steaming them at 100 °C for 15 minutes. Once back at room temperature, they were rinsed and immersed in phosphate buffered saline with KCI (K-PBS) + tween 20 (137 mM NaCl, 2.7 mM KCI, 0.1% Tween20) for five minutes followed by K-PBS. Afterwards, the slides were transferred to Shandon racks (Thermo Scientific, USA). First, they were blocked with Peroxidase-Blocking Solution (Dako; S2023) for 10 minutes, and then with bovine serum albumin, BSA, (2% BSA in K-PBS, Sigma Aldrich; A79096) for 20 minutes. Primary antibodies were diluted in BSA and incubated overnight at 4°C. The following day, the slides were incubated with HRP (horseradish peroxidase)-labeled polymer conjugated to secondary antibody (EnVision + System-HRP Labeled Polymer) anti-rabbit for 40 minutes at room temperature. This was followed by incubation with DAB (Liquid DAB + Substrate System TM, Dako; K3468) for five minutes. Hematoxylin was used as counterstaining. The slides were finally mounted using an automated glass coverslipper (Dako). Images were captured on Zeiss Axio Scan.Z1.

### Statistics and data analysis

The Kaplan-Meier method was used to create the survival curves, and they were compared through the log-rank test. The maximum temperatures on tumor surface were compared using one-way ANOVA with Tukey's multiple comparisons test. The fluorescence emitted by ICG measured by the Fluobeam before and after PTT was compared using two-way ANOVA with Sidak's multiple comparisons test. The data was plotted in Prism7 and shown as mean ± SEM.

### Example 1

### Photothermal abilities of ICG-Glu-Glu-AE105 and unconjugated ICG in vitro

The uPAR-targeted optical probes ICG-Glu-Glu-AE105 were used in all Examples. It was made by the method disclosed in WO 2016/041558.

In order to test the ability of ICG-Glu-Glu-AE105 to heat under NIR light, different concentrations of the compound in solution were placed in a plastic cuvette under a laser, at an intensity of 2 W/cm2. The samples were then irradiated for ten minutes and the temperatures detected with a FLIR camera. For the highest concentration tested, 0.5 mg/ml, temperature increased fast in the first minutes and slower and in a more steady way after that, reaching a maximum temperature of 70.8 ± 3 °C after ten minutes of irradiation.

In contrast, lower concentrations reached their maximum peak fast; at five minutes for 0.1mg/ml (62.7 ± 1.6 °C), and around two to three minutes for 0.05 and 0.025 mg/ml (51.4 ± 0.9 and 40.7 ± 0.4 °C respectively). From that point, temperatures started to decrease most likely due to the irreversible degradation of ICG, which impedes heat generation (Wang et al., 2013).

Additionally, unconjugated ICG was also tested for its photothermal abilities in vitro to confirm that uPAR conjugation did not hinder the heat generation process. Different concentrations of ICG in solution were irradiated as it was done for the conjugated dye. The highest concentration tested was 0.1 mg/ml of ICG, which showed a maximum temperature of 71.4 ± 1.1 °C after ten minutes. As expected, the maximum temperature was very similar to the one reached by 0.5 mg/ml of ICG-Glu-Glu-AE105, because a concentration of 0.5 mg/ml of ICG-Glu-Glu-AE105 corresponds to approximately 0.18 mg/ml of ICG alone. Lower concentrations of ICG such as 0.05 mg/ml and 0.025 mg/ml showed early peaks and lower maximum temperatures. 0.05 mg/ml of ICG reached a maximum of 68.8 ± 0.7 °C after seven minutes of irradiation, meanwhile 0.025 mg/ml of ICG peaked at three minutes at 50 ± 0.2 °C.

### Example 2

### ICG-Glu-Glu-AE105 and unconjugated ICG accumulation in vivo

After confirming the ability of ICG-Glu-Glu-AE105 to heat in vitro at different concentrations, we proceeded to test the accumulation of the compound in a subcutaneous mouse model of glioblastoma. For this, mice bearing 300 or 600 mm³ U-87MG.luc2 tumors were injected with either 46 or 23 nmol of ICG-Glu-Glu-AE105 in 0.2 ml intravenously. To quantify the accumulation of the ICG-linked agent in the tumor at different time points, the animals were scanned with the Fluobeam camera, a fluorescence imaging device which detects the signal from the ICG molecules throughout the body.

The accumulation of ICG-Glu-Glu-AE105 was highly localized in the tumor and as shown in Figure 1 accumulation reached a peak at around four hours after injection. As expected, higher concentrations of ICG-Glu-Glu-AE105 showed higher levels of accumulation. Interestingly, there were no size-dependent differences in ICG-Glu-Glu-AE105 accumulation for the two tumor volumes included in the study. 300 mm³ was the size chosen for the rest of the *in vivo* studies described in these Examples.

In order to show that only ICG linked to the uPAR agonist and not ICG alone can gather significantly in the tumor at low concentrations, unconjugated ICG was also studied for its accumulation in the tumors at different timepoints. A concentration of 46 nmol of ICG was injected intravenously, and the ICG distribution followed in the mice. It was observed that rather few ICG molecules found their way to the tumor. Most likely, the rest of the injected dose ended up in spleen and liver. This confirmed that the AE105 peptide allows for a specific accumulation of ICG in the tumor. Injecting much higher concentrations of unconjugated ICG would be needed to see a significant signal in the tumor when AE105 is not present.

### Example 3

### Ability of ICG-Glu-Glu-AE105 to ablate tumors in vivo

After determining ideal dose and treatment schedule, different groups of mice were set up in order to test the ability of ICG-Glu-Glu-AE105 as a photothermal agent, capable of specifically ablating tumors in vivo.

For this, mice were injected subcutaneously with U-87 MG.luc2 cells, a type of glioblastoma cells expressing luciferase, which allows for bioluminescence imaging when luciferin is injected. The bioluminescence signal serves as a way to determine treatment success and follow tumor growth. When tumors reached ^{∼}300 mm³, five groups were set up; ICG-Glu-Glu-AE105 group (n = 6, animals receiving ICG-Glu-Glu-AE105 and laser treatment), ICG group (n = 4, animals receiving unconjugated ICG and laser treatment), saline group (n = 4, animals receiving saline and laser treatment), sham group (n = 5, animals receiving ICG-Glu-Glu-AE105 but no laser treatment) and a control group (n = 4, animals did not receive anything and did not undergo laser treatment). The timeline for the experiments can be seen on Figure 2. On baseline (day -1), mice underwent a pre-treatment bioluminescence scan. Following, on treatment day (day 0), mice were injected with either ICG-Glu-Glu-AE105, ICG alone, saline or not injected as it was the case for the control group. Approximately four hours after injection, all groups but sham and control were irradiated for 5 minutes with NIR light at 2 W/cm2, and the temperatures on tumor surface were measured with a FLIR camera. Sham and control mice were placed under the laser beam for 5 minutes with the laser turned off to imitate the process. After treatment, all mice underwent a post-treatment bioluminescence scan. Additionally, ICG-Glu-Glu-AE105 and sham groups were fluorescence-scanned with the Fluobeam before and after PTT. All mice were bioluminescence-imaged every other day after treatment and tumors measured with a caliper until they reached 1000 mm³, when the mice were euthanized.

### 3.1 Temperature increase on the tumor surface during PTT

When tumors from the ICG-Glu-Glu-AE105 group were irradiated, surface temperatures increased at a quick pace and after five minutes the highest temperature reached was around 52 °C, compared to around 45 °C for ICG and saline groups, which most likely reflects unspecific heating caused by the laser alone. Sham and control tumors were placed under the laser but were not irradiated, thus showing basal tumor temperatures. The high temperatures reached by the ICG-Glu-Glu-AE105 group and not by the unconjugated ICG group prove the high specificity of the uPAR agonist, which leads to high accumulation of ICG-Glu-Glu-AE105 in the tumor and therefore allows for significant tumor death through localized heating.

Figure 3 shows the maximum temperatures reached on the tumor surface for the different groups. Data shown is mean ± SEM.

### 3.2 Tumor growth and survival after ICG-Glu-Glu-AE105-based PTT

The high temperatures reached by the ICG-Glu-Glu-AE105 group correlated with the delay in tumor growth that the group presented when compared to all the other groups (Fig. 4a-e). Accordingly, survival was extended and one mouse even experienced complete tumor disappearance up until 60 days after PTT, when the study was terminated (Fig. 4f). No significant differences in tumor growth or survival were detected among all the other control groups. The median survival was 13.5 days for ICG-Glu-Glu-AE105 group, 10 days for the ICG group, 6 days for the saline group, 8 days for the sham group and 9 days for the control group.

Figure 4a-e) show the growth curves from the ICG-Glu-Glu-AE105 (n = 6), ICG (n = 4), Saline (n = 4), Sham (n = 5) and Control (n = 4) groups respectively. Curves stopped on day 29 as only one ICG-Glu-Glu-AE105 mouse was left. Figure 4 f) shows the survival curves for the different groups. The shortest survival was observed for the control group, followed by the ICG and saline groups, followed by the sham group whereas the longest survival was observed in the ICG-Glu-Glu-AE105 group.

### 3.3 Bioluminescence signal after PPT

In line with the results found for tumor growth and survival after PTT for the different groups, the bioluminescence imaging after treatment showed a reduced signal for the ICG-Glu-Glu-AE105 group due to tumor death caused by PTT. This signal was maintained throughout the study and stayed significantly lower than all the other groups as shown in Figure 5A.

Finally, fluorescence scans were performed to confirm the ICG degradation in the ICG-Glu-Glu-AE105 molecules after irradiation at high laser intensity, as already observed in vitro. As expected, the ICG tumor signal for the ICG-Glu-Glu-AE105 group was reduced significantly after PTT, which did not happen for nonirradiated sham tumors. This result is shown in Figure 5B.

### Example 4

### Tumor response tested ex vivo

In order to study tumor response *ex vivo,* tumors from n = 2 mice per group were extracted two days after PTT. Hematoxylin and eosin staining, uPAR and CD68 staining were performed. Relevant information was extracted from this analysis; as expected, ICG-Glu-Glu-AE105 showed a high degree of cell death when compared to all of the other groups, as depicted through the H&E staining. As for uPAR, it is known to be overexpressed in this tumor type. This can be confirmed throughout all tumor tissue for all the groups, except for ICG-Glu-Glu-AE105 due to tumor death caused by PTT. Finally, macrophages were detected through CD68 staining to observe co-localization with uPAR signal, which could be noted, again, for all viable tumor regions.

### References

Chatterjee, D. K., Diagaradjane, P. & Krishnan, S. Nanoparticle-mediated hyperthermia in cancer therapy. Ther. Deliv. 2, 1001-1014 (2011).
Day, E. S. et al. Nanoshell-mediated photothermal therapy improves survival in a murine glioma model. J. Neurooncol. 104, 55-63 (2011).
Riley, R. S. & Day, E. S. Gold nanoparticle-mediated photothermal therapy: applications and opportunities for multimodal cancer treatment. Wiley Interdiscip. Rev. Nanomedicine Nanobiotechnology 9, e1449 (2017).
Jaque, D. et al. Nanoparticles for photothermal therapies. Nanoscale 6, 9494-530 (2014).
Boni, L. et al. Clinical applications of indocyanine green (ICG) enhanced fluorescence in laparoscopic surgery. Surg. Endosc. 29, 2046-2055 (2015).
Shirata, C. et al. Near-infrared photothermal/photodynamic therapy with indocyanine green induces apoptosis of hepatocellular carcinoma cells through oxidative stress. Sci. Rep. 7, 13958 (2017).
Wang, H. et al. Indocyanine green-incorporating nanoparticles for cancer theranostics. Theranostics 8, 1227-1242 (2018).
Wang, Y. et al. Tumor-Penetrating Nanoparticles for Enhanced Anticancer Activity of Combined Photodynamic and Hypoxia-Activated Therapy. ACS Nano 11, 2227-2238 (2017).
Yan, F. et al. Molecular imaging-guided photothermal/photodynamic therapy against tumor by iRGD-modified indocyanine green nanoparticles. J. Control. Release 224, 217-228 (2016).
Montuori, N. et al. Urokinase type plasminogen activator receptor (uPAR) as a new therapeutic target in cancer. Transl Med UniSa 15, 15-21 (2016).
Li, D., Liu, S., Shan, H., Conti, P. & Li, Z. Urokinase plasminogen activator receptor (uPAR) targeted nuclear imaging and radionuclide therapy. Theranostics 3, 507-515 (2013).
Juhl, K., Christensen, A., Persson, M., Ploug, M. & Kjaer, A. Peptide-Based Optical uPAR Imaging for Surgery: In Vivo Testing of ICG-Glu-Glu-AE105. PLoS One 11, e0147428 (2016).
Wang, Y.-G., Kim, H., Mun, S., Kim, D. & Choi, Y. Indocyanine green-loaded perfluorocarbon nanoemulsions for bimodal (19)F-magnetic resonance/nearinfrared fluorescence imaging and subsequent phototherapy. Quant. Imaging Med. Surg. 3, 132-13240 (2013).
Deng, Z., Liu, C., Tao, W. & Zhu, D. Improvement of skin optical clearing efficacy by topical treatment of glycerol at different temperatures. J. Phys. Conf. Ser. 277, 012007 (2011).
Vargas, G., Chan, E. K., Barton, J. K., Rylander, H. G. & Welch, A. J. Use of an agent to reduce scattering in skin. Lasers Surg. Med. 24, 133-141 (1999).
Simón, M., Nørregaard, K., Jørgensen, J. T., Oddershede, L. B. & Kjaer, A. Fractionated photothermal therapy in a murine tumor model: comparison with single dose. Int. J. Nanomedicine Volume 14, 5369-5379 (2019).

## Claims

1. Probe having the formula X-Y-Z and pharmaceutically acceptable salts thereof, wherein
X represents a photothermic agent capable of absorbing light that is transformed into heat upon irradiation with an external source of light and capable of being detected either directly or indirectly in an optical imaging procedure;
Y represents a spacer or is absent;
and Z is a uPAR targeting peptide;
for use as a medicament subject to irradiation with an external laser source that activates X.

2. Probe having the formula X-Y-Z and pharmaceutically acceptable salts thereof, wherein
X represents a photothermic agent capable of absorbing light that is transformed into heat upon irradiation with an external laser source and capable of being detected either directly or indirectly in an optical imaging procedure;
Y represents a spacer or is absent;
and Z is a uPAR targeting peptide;
for use in treatment of cancer subject to irradiation with an external laser source that activates X.

3. Probe according to claim 1 or 2 wherein X is a fluorescent agent.

4. Probe according to claim 3 wherein the fluorescent agent is selected from the NIR-I group fluorophores comprising: ICG, Methylene blue, 5-ALA, Protoporphyrin IX, IRDye800CW, ZW800-1, Cy5, Cy7, Cy5.5, Cy7.5, IRDye700DX, Alexa fluor 488, Fluorescein isothiocyanate or wherein the fluorescent agent is selected from the NIR-II group fluorophores comprising: Flav7, CH1055, Q1, Q4, H1, IR-FEP, IR-BBEP, IR-E1, IR-FGP, IR-FTAP.

5. Probe according to any of claims 1 to 4 wherein Y is Glu-Glu or Glu-Glu-O2Oc-O2Oc.

6. Probe according to any of claims 1 to 5 wherein Z is selected from (D-Asp)-([beta]-cyclohexyl-L-alanine)-(Phe)-(D-Ser)-(D-Arg)-(Tyr)-(Leu)-(Trp)-(Ser) or Asp-Cha-Phe-ser-arg-Tyr-Leu-Trp-Ser-NH2, wherein Cha is β-cyclohexyl-L-alanine.

7. Probe according to claims 1 or 2 is selected from the group comprising the probes wherein X is ICG and Y is Glu-Glu and Z is (D-Asp)-([beta]-cyclohexyl-L-alanine)-(Phe)-(D-Ser)-(D-Arg)-(Tyr)-(Leu)-(Trp)-(Ser); X is ICG and Y is Glu-Glu and Z is Asp-Cha-Phe-ser-arg-Tyr-Leu-Trp-Ser-NH2, wherein Cha is β-cyclohexyl-L-alanine; X is ICG and Y is Glu-Glu-O2Oc-O2Oc and Z is (D-Asp)-([beta]-cyclohexyl-L-alanine)-(Phe)-(D-Ser)-(D-Arg)-(Tyr)-(Leu)-(Trp)-(Ser); X is ICG and Y is Glu-Glu-O2Oc-O2Oc and Z is Asp-Cha-Phe-ser-arg-Tyr-Leu-Trp-Ser-NH2, wherein Cha is β-cyclohexyl-L-alanine.

8. Probe according to any of claims 1 to 7 wherein the cancer is selected from the group comprising: glioblastoma, brain cancer, oropharyngeal cancer, head and neck cancer, prostate cancer, breast cancer, gastro-intestinal cancer, colorectal cancer (CRC), lung cancer, non-small lung cancer (NSCLC).

9. Probe according to any of claims 1 to 8 for use in the treatment of cancer in combination with optical imaging of X or for use in the treatment of cancer in combination with surgery based on the optical imaging of X.

10. Probe according to any of claims 1 to 9 for use in the treatment of cancer in combination with surgery based on positron emitting topography.

11. Probe according to any of claims 1 to 10 wherein the probe is administered to a subject in a dose of 0.1 - 1000 mg per 70 kg.

12. Method of treating cancer comprising
(vii) administering a probe having the formula X-Y-Z or pharmaceutically acceptable salts thereof, wherein
X represents a photothermic agent capable of absorbing light that is transformed into heat upon irradiation with an external source of light and capable of being detected either directly or indirectly in an optical imaging procedure;
Y represents a spacer or is absent;
and Z is a uPAR targeting peptide;
to a subject
(viii) subjecting the subject to irradiation with an external laser source that activates X.

13. Method according to claim 12 wherein the X is a fluorescent probe being activated by near infrared light.

14. Method according to claim 13 wherein the probe is selected from the group comprising the probes wherein X is ICG and Y is Glu-Glu and Z is (D-Asp)-([beta]-cyclohexyl-L-alanine)-(Phe)-(D-Ser)-(D-Arg)-(Tyr)-(Leu)-(Trp)-(Ser); X is ICG and Y is Glu-Glu and Z is Asp-Cha-Phe-ser-arg-Tyr-Leu-Trp-Ser-NH2, wherein Cha is β-cyclohexyl-L-alanine; X is ICG and Y is Glu-Glu-O2Oc-O2Oc and Z is (D-Asp)-([beta]-cyclohexyl-L-alanine)-(Phe)-(D-Ser)-(D-Arg)-(Tyr)-(Leu)-(Trp)-(Ser); X is ICG and Y is Glu-Glu-O2Oc-O2Oc and Z is Asp-Cha-Phe-ser-arg-Tyr-Leu-Trp-Ser-NH2, wherein Cha is β-cyclohexyl-L-alanine.

15. Method according to claim 12 further comprising one or more additional steps carried out either before or after the method according to claim 12:
(ix) optical imaging of X;
(x) optical guided surgery based on the imaging of X;
(xi) optical guided surgery based on PET imaging of a uPAR directed target;
(xii) identification and diagnostics based on PET imaging of a uPAR directed target.
